# EUROPÄISCHE PATENTSCHRIFT

(11) **EP 2 281 793 B1**
(45) Veröffentlichungstag und Bekanntmachung des Hinweises auf die Patenterteilung: **30.09.2015**
(21) Anmeldenummer: 09014343.9
(22) Anmeldetag: 17.11.2009
(51) Int. Cl.: C07C 29/152, C07C 31/04, B01D 53/00

(54) **Verfahren und Anlage zur Herstellung von Methanol**
Method and device for manufacturing methanol
Procédé et installation destinés à la fabrication de méthanol

(30) Priorität: 23.07.2009 DE 102009034551
(43) Veröffentlichungstag der Anmeldung: 09.02.2011
(73) Patentinhaber: Air Liquide Global E&C Solutions Germany GmbH, 60439 Frankfurt am Main (DE)
(72) Erfinder: Bormann, Andreas, Dr., 60389 Frankfurt a. M. (DE); Gronemann, Veronika, 61184 Karben (DE); Morgenroth, Rainer, 61381 Friedrichsdorf (DE); Hagemann, Jan, 35390 Gießen (DE); Hackel, Philipp, Marius, Dr., 61250 Usingen (DE); Schlichting, Holger, Dr., 65719 Hofheim (DE)

(56) Entgegenhaltungen:
- WO-A1-2009/030353
- WO-A2-03/042144

## Beschreibung

Die Erfindung betrifft ein Verfahren zur Herstellung von Methanol sowie eine Anlage zur Durchführung dieses Verfahrens. Insbesondere betrifft die Erfindung ein Verfahren zur Umsetzung von Synthesegas mit hohem Anteil an Inertkomponenten zu Methanol. Die Erfindung betrifft ferner ein Verfahren zur Umrüstung einer bestehenden Anlage zur Herstellung von Methanol vom Betrieb mit inertenarmem Synthesegas für den Betrieb mit inertenreichem Synthesegas.

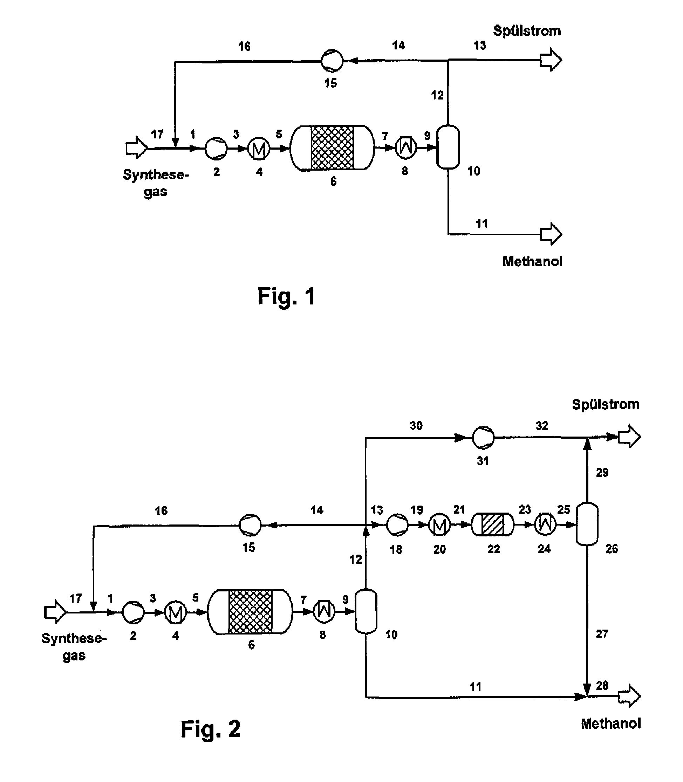

Verfahren zur Herstellung von Methanol durch katalytische Umsetzung von Wasserstoff und Kohlenstoffoxide enthaltendem Synthesegas sind der Fachwelt seit langer Zeit bekannt. So wird in Ullmann's Encyclopedia of Industrial Chemistry, Sixth Edition, 1998 Electronic Release, Kapitel "Methanol", Unterkapitel 5.2 "Synthesis" ein Verfahren zur Herstellung von Methanol beschrieben, das schematisch und vereinfacht in Fig. 1 dargestellt ist. Dort wird ein Wasserstoff und Kohlenstoffoxide enthaltender Synthesegasstrom über Leitung 1 einem Verdichter 2 zugeführt und von diesem auf den Reaktionsdruck von typischerweise 5 bis 10 MPa gebracht. Verdichter 2 und Verdichter 15 können dabei technisch miteinander gekoppelt sein. Der verdichtete Synthesegasstrom wird über Leitung 3 einem Wärmetauscher 4 zugeführt und in diesem auf die Reaktionstemperatur gebracht, wobei zumeist der Wärmetausch gegen den heißen Produktgasstrom aus dem Synthesereaktor erfolgt (nicht dargestellt in Fig. 1). Der vorgeheizte Synthesegasstrom tritt über Leitung 5 in den Synthesereaktor 6 ein, wo bei Temperaturen zwischen 200 und 300 °C die teilweise Umsetzung von Wasserstoff mit Kohlenoxiden an einem Methanolsynthesekatalysator erfolgt, wobei ein Produktgemisch erhalten wird, das Synthesegas enthält. Über Leitung 7 wird das Produktgemisch aus dem Synthesereaktor abgeführt. Nach Abkühlung im Wärmetauscher 8 gelangt das Produktgemisch über Leitung 9 in den Abscheider 10, wo Methanol als Rohmethanol abgetrennt und Ober Leitung 11 der weiteren Produktaufarbeitung zugeführt wird. Das im Abscheider erhaltene Gasprodukt wird über Leitung 12 abgeführt und in einen Spülstrom, der über Leitung 13 abgeführt wird, und einen Kreislaufstrom, der über Leitung 14 dem Kreislaufverdichter 15 zugeführt wird, aufgetrennt. Über den Spülstrom werden Inertkomponenten aus dem Verfahren ausgeschleust. Über Leitung 16 wird der Kreislaufstrom zum Synthesereaktor 6 zurückgeführt, wobei über Leitung 17 frisches Synthesegas herangeführt und mit dem Kreislaufstrom vereinigt wird. Das Verhältnis der Stoffmengenströme von Kreislaufstrom zu Frischgasstrom wird als Kreislaufverhältnis bezeichnet.

Ein moderneres, zweistufiges Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom aus dem Verfahren entfernt, um zu verhindern, dass sich Inertkomponenten innerhalb des Synthesekreislaufes anreichem. Diese Maßnahme wird auch in der deutschen Offenlegungsschrift DE 2934332 A1 und der europäischen Patentanmeldung EP 1016643 A1 gelehrt.

Nachteilig ist bei den beiden oben beschriebenen Verfahren, dass bei der Verarbeitung von Synthesegasen mit hohem Anteil an Inertkomponenten das Kreislaufverhältnis erhöht werden muss, da aufgrund der geringeren Partialdrücke der Reaktanden der Umsatz zu Methanol pro Durchgang durch den Synthesereaktor geringer ist als bei inertenarmem Synthesegas. Dies führt zu einer Erhöhung der benötigten Verdichterleistung, sowie - bei vorgegebener Produktionskapazität für Methanol - zu größeren Dimensionen für Apparate und Rohrleitungen.

Als lnertkomponenten werden dabei einerseits anorganische Gasbestandteile wie Stickstoff oder Edelgase verstanden, die beispielsweise aus der Erzeugung von Synthesegas ausgehend von Erdgas mit entsprechenden Bestandteilen erhalten werden. Solche Erdgase werden beispielsweise aus asiatischen Lagerstätten gewonnen. Andererseits wird auch nicht umgesetztes Methan, das bei der Vergasung von Erdgas im Synthesegasprodukt enthalten sein kann, als ein im Sinne der Methanolsynthese inertes Gas betrachtet. Ferner wird ein stickstoffhaltiges Synthesegas erhalten, wenn die Vergasung von Erdgas mit Luft oder sauerstoffangereicherter Luft durchgeführt wird, wie es beispielsweise in der internationalen Patentanmeldung WO 96/14279 A1 vorgeschlagen wird.

Die Problematik der Verarbeitung inertenreichen Synthesegases in der Methanolsynthese ist seit längerem bekannt. Es wurden bereits verschiedene technische Lösungsansätze vorgeschlagen, die sich aber aufgrund ihrer Nachteile nicht durchgesetzt haben.

So wird in der Offenlegungsschrift DE 1296133 B vorgeschlagen, das Inertkomponenten wie Stickstoff, Methan oder Argon enthaltende Rohsynthesegas durch eine Xylolwäsche zu behandeln, wodurch deutliche Absenkungen der Gehalte der Inertkomponenten erzielt werden sollen. Nachteilig ist hier, dass die Temperatur des Synthesegases vor Eintritt in die Gaswäsche auf -10 bis -30 °C abgesenkt werden muss, um die Partialdrücke der Inertkomponenten signifikant abzusenken. Damit entsteht ein hoher Exergieverlust. Zudem fällt ein beladenes Absorptionsmittel an, das nachbehandelt werden muss und das die der Methanolsynthese verfahrensfremde Komponente Xylol enthält.

Eine ähnliche technische Lehre ist der deutschen Offenlegungsschrift DE 10156092 A1 zu entnehmen, in der vorgeschlagen wird, jedem katalytischen Reaktionssystem zur Herstellung von Methanol eine Absorptionsstufe vorzuschalten, welche als Absorptionsmittel Methanolsynthesekatalysator enthält, und welche bei einer Temperatur betrieben wird, die unterhalb der für die katalytische Umsetzung zu Methanol liegt. Als Absorptionsmittel wird hierbei ein verfahrenseigener Hilfsstoff herangezogen wird, allerdings bleiben die beiden bereits ausgeführten Nachteile einer benötigten Temperaturabsenkung sowie einer Nachbehandlung oder Entsorgung des Absorptionsmittels weiterhin bestehen.

Ein Verfahren zur Herstellung von Methanol aus einem aus der autothermen Vergasung von Erdgas erhaltenen Synthesegas, enthaltend jeweils 20 bis 50 % Wasserstoff, Kohlenmonoxid und Methan, beschreibt die Patentanmeldung EP 1819653 A1. Hierbei werden keine besonderen Maßnahmen bezüglich des im Reaktorprodukt des Methanolsynthesereaktors verbleibenden Methans ergriffen, sondern der Wasserstoffgehalt mittels Konvertierung erhöht, sodann der Wasserstoff - gegebenenfalls mit dem ebenfalls erzeugten Kohlendioxid - abgetrennt und dem Methanolsynthesereaktor wieder aufgegeben. Diese Maßnahme erhöht zwar die Partialdrücke der Reaktanden, der Kreislaufstrom bleibt allerdings durch den hohen Inertkomponentenanteil hoch.

Insgesamt ist daher festzustellen, dass bislang keine befriedigende technische Lösung der Aufgabenstellung gefunden wurde, obwohl das Problem, wie dargestellt, bereits seit längerer Zeit besteht. Viele der oben diskutierten Verfahren zielen zudem darauf ab, den Inertkomponenten enthaltenden Spülstrom durch eine entsprechende Behandlung möglichst zu verkleinern.

Aufgabe der vorliegenden Erfindung ist es daher, die oben genannten Nachteile zu vermeiden und ein wirtschaftlicheres sowie technisch einfacher durchführbares Verfahren zur Herstellung von Methanol bei Verwendung von inertenreichem Synthesegas als Eduktgas bereitzustellen, das sich insbesondere durch geringen Energiebedarf, kleinere Apparatedimensionen und die Vermeidung prozessfremder Hilfsstoffe auszeichnet.

Diese Aufgabe wird bei einem Verfahren mit den Merkmalen (a) bis (e) des Anspruchs 1 im Wesentlichen dadurch gelöst, dass der Spülstrom einem Nachreaktor zugeführt wird, in dem ein weiterer Teil des Wasserstoffs und der Kohlenstoffoxide katalytisch zu Methanol umgesetzt werden, und aus dem erhaltenen, Synthesegas, Inertkomponenten und Methanoldampf enthaltenden Gemisch weiteres Methanol abgetrennt wird.

Der Spülstrom wird dazu verdichtet, mittels Wärmetauscher oder Erhitzer auf die Reaktionstemperatur gebracht und dem außerhalb des Synthesekreislaufs angeordneten Nachreaktor aufgegeben. Bei hinreichend kleinem Druckverlust über den Nachreaktor kann auch auf die zusätzliche Verdichtung verzichtet werden. Eine besonders wirtschaftliche Ausgestaltung des Verfahrens wird erreicht, wenn das Aufheizen des Spülstroms auf die Reaktionstemperatur durch Wärmetausch gegen das heiße Reaktionsgemisch vor Eintritt aus dem Synthesereaktor oder das heisse Produktgemisch nach Austritt aus dem Synthesereaktor erfolgt. Dadurch wird die Wärme im System gehalten und zur notwendigen Aufheizung des Gasstroms auf die erforderliche Reaktionstemperatur im Nachreaktor genutzt.

Im Nachreaktor erfolgt die Umsetzung eines weiteren Teils des im Spülstrom verbliebenen Wasserstoffs und der Kohlenoxide zu Methanol an einem für die Methanolsynthese aktiven Katalysator. Es können handelsübliche Methanolsynthesekatalysatoren auf Kupferbasis verwendet werden.

Gemäß einer bevorzugten Ausgestaltung der Erfindung ist vorgesehen, das Verhältnis von Kreislaufstrom zu Frischgasstrom (Kreislaufverhältnis) kleiner zu wählen als bei einem an sich bekannten Verfahren zur Herstellung von Methanol mit den Merkmalen (a) bis (e) des Anspruchs 1. Dies entspricht bei festgelegtem Frischgasstrom - gleichbedeutend einer festgelegten Synthesegas-Verarbeitungskapazität - einer Verkleinerung des Kreislaufstroms, und somit einer Verkleinerung der benötigten Apparate- und Leitungsdimensionen im Synthesekreislauf, sowie der benötigten Verdichterleistung. Zwar verschlechtert sich hierdurch die Raumzeitausbeute an Methanol im Synthesekreislauf; diese Verschlechterung wird aber überraschenderweise durch den zusätzlichen Synthesegas-Umsatz im Nachreaktor überkompensiert, da dort ein erheblich geringerer Synthesegas-Volumenstrom verarbeitet werden muss. Bevorzugt wird das Verfahren mit einem Verhältnis von Kreislaufstrom zu Frischgasstrom von 10 bis 90 %, besonders bevorzugt von 50 bis 80 % desjenigen eines Verfahrens zur Herstellung von Methanol mit den Merkmalen (a) bis (e) des Anspruchs 1 durchgeführt.

Vorteifhafterweise kann das Verhältnis von Kreislaufstrom zu Frischgasstrom (Kreislauf verhältnis) in Abhängigkeit von dem Anteil an Inertkomponenten im Frischgasstrom geregelt werden. Dies kann so erfolgen, dass bei Inbetriebnahme des Verfahrens bei festgelegtem Frischgasstrom zunächst ein hohes Kreislaufverhältnis gewählt wird. Es kann vorteilhaft sein, dabei den Nachreaktor mittels einer Bypassleitung ganz oder teilweise zu umgehen. Erst bei Aufbau einer signifikanten Konzentration an Inertkomponenten im Synthesekreislauf, die durch ein geeignetes Analyseverfahren nachgewiesen werden kann, wird das Kreislaufverhältnis reduziert und der dem Nachreaktor aufgegebene Spülstrom erhöht. Eine Nachführung des Kreislaufverhältnisses kann auch bei sukzessiver Desaktivierung des Katalysators im Synthesereaktor sinnvoll sein.

In Weiterbildung der Erfindung wird der nach Methanolabtrennung verbliebene, die Inertkomponenten sowie nicht umgesetztes Synthesegas enthaltende Gasstrom - gegebenenfalls nach Nachbehandlung - aus dem Verfahren entfernt. Bei hohem Anteil an Methan und noch signifikanten Anteilen an Wasserstoff und Kohlenstoffmonoxid kann der Gasstrom der weiteren stofflichen oder thermischen Nutzung, beispielsweise als Heizgas, zugeführt werden.

Gemäß einer bevorzugten Ausgestaltung der Erfindung wird das durch katalytische Umsetzung im Nachreaktor gewonnene Methanol separat oder, besonders bevorzugt, gemeinsam mit dem aus dem Produktgemisch des Synthesereaktors abgetrennten Methanol der weiteren Produktaufarbeitung zugeführt. Die Aufarbeitung des auf diese Weise gewonnenen Rohmethanols zu Reinmethanol erfolgt in an sich bekannter Weise durch mehrstufige Destillation bzw. Rektifikation.

Sowohl der oder die Synthesereaktoren als auch der Nachreaktor können wassergekühlt, gasgekühlt oder adiabat betrieben werden. In einer bevorzugten Ausführungsform des Verfahrens wird im Synthesekreislauf der erste Synthesereaktor wassergekühlt und der zweite Synthesereaktor gasgekühlt betrieben. Bei Verwendung von wassergekühlten Reaktoren kann der typischerweise erzeugte Mitteldruckdampf zu Kompressions- oder Heizzwecken verwendet werden.

Die Erfindung erstreckt sich auch auf eine Anlage zur Herstellung von Methanol aus einem Wasserstoff und Kohlenstoffoxide enthaltenden Synthesegas mit hohem Anteil an Inertkomponenten, die zur Durchführung des oben beschriebenen Verfahrens geeignet ist. Die Anlage umfasst einen oder mehrere Synthesereaktoren, in welchem ein Teil der Kohlenstoffoxide katalytisch zu Methanol umgesetzt wird, einen Abscheider zur Abtrennung des Methanols von dem Synthesegas, Leitungen zur Rückführung eines Synthesegas-Kreislaufstrom zum mindestens einen Synthesereaktor, Leitungen zum Abführen eines Spülstroms, und eine Leitung zum Zuführen eines Frischgasstroms an Synthesegas; sie ist gekennzeichnet durch einen mit einer Leitung zum Abführen des Spülstroms verbundenen Nachreaktor, in dem ein weiterer Teil des Wasserstoffs und der Kohlenstoffoxide katalytisch zu Methanol umgesetzt werden, und einen Abscheider zur Abtrennung weiteren Methanols aus dem im Nachreaktor erhaltenen, Synthesegas, Inertkomponenten und Methanoldampf enthaltenden Gemisch.

Die Erfindung betrifft ferner ein Verfahren zur Umrüstung einer bestehenden Anlage zur Herstellung von Methanol nach einem Verfahren gemäß Merkmal (a) bis (e) des Anspruchs 1 vom Betrieb mit inertenarmem Synthesegas für den Betrieb mit inertenreichem Synthesegas, insbesondere für die Durchführung des oben beschriebenen, erfindungsgemäßen Verfahrens nach erfolgter Umrüstung. Das Verfahren zur Umrüstung der Anlage ist **dadurch gekennzeichnet, dass** dem Spülstrom ein Nachreaktor nachgeschaltet wird, aus dessen Produktgemisch weiteres Methanol abgetrennt wird und dass die Anlage mit einem Verhältnis von zurückgeführtem Synthesegasstrom zum Frischgasstrom betrieben wird, das kleiner ist als beim Betrieb der Anlage mit inertenarmem Synthesegas.

Weiterbildungen, Vorteile und Anwendungsmöglichkeiten der Erfindung ergeben sich auch aus der nachfolgenden Beschreibung von Ausführungsbeispielen und der Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigen
- Figur 1 schematisch eine Anlage zur Herstellung von Methanol nach einem Verfahren gemäß Merkmal (a) bis (e) des Anspruchs 1 (Stand der Technik); diese wurde bereits oben in der Beschreibungseinleitung beschrieben,
- Figur 2 schematisch eine Anlage zur Herstellung von Methanol gemäß einer bevorzugten Ausführungsform der Erfindung.

In der in Figur 2 dargestellten Anlage wird ein Gemisch aus frischem und zurückgeführtem Synthesegas durch eine Leitung 1 einem Verdichter 2 zugeführt und von diesem auf den Reaktionsdruck von typischerweise 5 bis 10 MPa gebracht. Der verdichtete Synthesegasstrom wird über Leitung 3 einem Wärmetauscher 4 zugeführt und in diesem auf die Reaktionstemperatur gebracht, wobei der Wärmetausch bevorzugt gegen den heißen Produktgasstrom aus dem Synthesereaktor erfolgt (nicht dargestellt in Fig. 2). Der vorgeheizte Synthesegasstrom tritt über Leitung 5 in den Synthesereaktor 6 ein, wo bei Temperaturen zwischen 200 und 300 °C die teilweise Umsetzung von Wasserstoff mit Kohlenoxiden (Synthesegas) an einem Methanolsynthesekatalysator erfolgt, wobei ein Produktgemisch erhalten wird, das Methanol, Wasser und Synthesegas enthält. Als Methanolsynthesekatalysator wird ein kommerziell erhältlicher Katalysator auf Kupferbasis verwendet. Die Raumgeschwindigkeit im Synthesereaktor beträgt typischerweise 10000 bis 30000 h⁻¹. Über Leitung 7 wird das Produktgemisch aus dem Synthesereaktor abgeführt. Nach Abkühlung im Wärmetauscher 8 gelangt das Produktgemisch über Leitung 9 in den Abscheider 10, wo Methanol als Rohmethanol abgetrennt und über Leitungen 11 und 28 der weiteren Produktaufarbeitung zugeführt wird. Diese kann in an sich bekannter, aber nicht in Fig. 2 dargestellter Weise durch Destillation oder Rektifikation erfolgen. Das im Abscheider erhaltene Gasprodukt wird über Leitung 12 abgeführt und in einen Spülstrom, der über Leitung 13 abgeführt wird, und einen Kreislaufstrom, der über Leitung 14 dem Kreislaufverdichter 15 zugeführt wird, aufgetrennt. Über Leitung 16 wird der Kreislaufstrom zum Synthesereaktor 6 zurückgeführt, wobei über Leitung 17 frisches Synthesegas herangeführt und mit dem Kreislaufstrom vereinigt wird.

Der über Leitung 13 abgeführte Spülstrom wird über einen Verdichter 18 auf einen Reaktionsdruck von 0.5-2 MPa oberhalb des mittleren Reaktionsdrucks des Synthesekreislaufs verdichtet. Der Spülstrom wird anschließend über Leitung 19 einem Wärmetauscher 20 zugeführt und auf eine Reaktoreintrittstemperatur von 190 bis 250 °C aufgeheizt. Über Leitung 21 wird er dem Nachreaktor 22 zugeführt. Im Nachreaktor wird ebenfalls ein kommerziell erhältlicher Katalysator auf Kupferbasis als Methanolsynthesekatalysator verwendet. Die Raumgeschwindigkeit im Nachreaktor beträgt typischerweise 5000 bis 15000 h⁻¹. Das Produktgemisch tritt aus dem Reaktor aus und wird über Leitung 23 dem Wärmetauscher 24 zugeführt, wo eine Abkühlung auf Temperaturen deutlich unterhalb des Taupunktes für Methanol und Wasser, bevorzugt zwischen 30 und 60 °C, erfolgt. Nach Abkühlung im Wärmetauscher 24 gelangt das Produktgemisch über Leitung 25 in den Abscheider 26, wo Methanol als Rohmethanol abgetrennt und über Leitung 27 gemeinsam mit dem über Leitung 11 herangeführten Methanolprodukt des Synthesereaktors der weiteren Produktaufarbeitung über Leitung 28 zugeführt wird. Das im Abscheider erhaltene Gasprodukt wird über Leitung 29 abgeführt und nach optionaler Nachbehandlung der an sich bekannten, aber nicht in Fig. 2 dargestellten stofflichen oder energetischen Nutzung zugeführt oder verworfen.

Beim Betrieb der in Fig. 2 dargestellten Anlage mit Nachreaktor wird das Verhältnis von Kreislaufstrom zu Frischgasstrom (Kreislaufverhältnis) des Synthesekreislaufs im Vergleich zum Betrieb der Anlage ohne Nachreaktor abgesenkt. Bevorzugt liegt das Kreislaufverhältnis beim Betrieb mit Nachreaktor im Bereich von 10 bis 90 % desjenigen beim Betrieb der Anlage ohne Nachreaktor. Besonders bevorzugt liegt das Kreislaufverhältnis beim Betrieb mit Nachreaktor im Bereich von 50 bis 80 % desjenigen beim Betrieb der Anlage ohne Nachreaktor.

Um einen Betrieb der Anlage zu ermöglichen, in dem der Nachreaktor mittels Bypass umgangen wird, kann das über Leitung 12 aus dem Abscheider 10 abgeführte Gasprodukt über Leitungen 30 und 32 aus dem Verfahren entfernt werden. Bevorzugt wird das über Leitung 32 abgeführte Gasprodukt vor der Entfernung aus dem Verfahren mit dem in Leitung 29 abgeführten Gasprodukt vereinigt. Eine gegebenenfalls benötigte Druckerhöhung kann über Verdichter 31 realisiert werden.

In Fig. 2 sind in den Leitungswegen 13, 19, 21 zum Nachreaktor und im Leitungsweg 30, 32 der Bypassleitung Verdichter dargestellt. Bei geeigneten Druckverhältnissen kann aber auch einer oder beide dieser Verdichter gegen Regelventile oder Gebläse ersetzt werden, um geeignete Mengenströme zum Nachreaktor und/oder zum Bypass einzustellen.

Bevorzugt wird beim Betrieb der Anlage mit Nachreaktor der gesamte Spülstrom über Leitungen 13, 19, 21 zum Nachreaktor geführt, um die Methanolausbeute zu optimieren. Es kann jedoch sinnvoll sein, bei besonderen Betriebszuständen der Anlage wie beispielsweise bei An- und Abfahrvorgängen nur einen Teil des Spülstroms über Leitungen 13, 19, 21 zum Nachreaktor zu führen und den restlichen Spülstrom über Leitungen 30, 32 aus dem Verfahren zu entfernen. In Abhängigkeit von den Verfahrensbedingungen können diese Anteile variabel gewählt werden.

Bei geplanter stofflicher oder energetischer Nutzung des Spülstroms kann es vorteilhaft sein, das über die Leitungen 30, 32 im Bypass um den Nachreaktor geführte Gas nicht mit dem Gasprodukt aus Abscheider 26 zusammenzuführen, sondern es separat zu verarbeiten, da sein Gehalt an Wasserstoff und Kohlenstoffmonoxid in der Regel höher ist als beim letztgenannten Gasprodukt.

Mit der Erfindung wird somit ein wirtschaftliches Verfahren zur Herstellung von Methanol vorgeschlagen, das sich dadurch auszeichnet, dass auch Synthesegase mit hohen Anteilen an Inertkomponenten verarbeitet werden können. Im Gegensatz zu den im Stand der Technik vorgeschlagenen Verfahren zeichnet sich das erfindungsgemäße Verfahren durch die Abwesenheit prozessfremder oder entsorgungs- bzw. regenerierungsbedürftiger Stoffe wie Absorptionsmittel aus. Als weitere Vorteile sind apparative Einfachheit, kleine Apparategrößen und Rohrieitungsdimensionen; Einsparungen an Katalysator sowie ein geringerer Energiebedarf, beispielsweise für die benötigte Verdichterarbeit, zu nennen.

### Zahlenbeispiele

Zur Verdeutlichung der Wirtschaftlichkeit und technischen Durchführbarkeit des Verfahrens dienen die folgenden Beispiele, die die Vorteile des erfindungsgemäßen Verfahrens im Vergleich zum Stand der Technik verdeutlichen. Dabei werden die relativen Änderungen für wichtige Verfahrenskenngrößen angegeben, die sich bei Anwendung des erfindungsgemäßen Verfahrens im Vergleich zu einem Verfahren gemäß Stand der Technik mit gleicher Methanol-Produktionskapazität ergeben. Als Vergleichsverfahren dient im Beispiel 1 ein zweistufiges Verfahren zur Methanolherstellung mit einem ersten, wassergekühlten und einem zweiten, gasgekühlten Methanolsynthesereaktor, wie es die EP 0 790 226 B1 lehrt; das dort beschriebene Herstellungsverfahren wird durch Bezugnahme ausdrücklich in die vorliegende Beschreibung aufgenommen. Im Beispiel 2 dient ein einstufiges Verfahren zur Methanolsynthese mit wassergekühltem Reaktor als Vergleichsverfahren, wie es beispielsweise die oben genannte Referenz "Ullmann's Encyclopedia of Industrial Chemistry" beschreibt.

### Beispiel 1: Synthesekreislauf mit zweistufigem Synthesereaktor (wasser- + gasgekühlt) + Nachreaktor (wassergekühlt)

| **Kenngröße** | **Relative Änderung / %** |
|---|---|
| Kreislaufverhältnis | -31,8 |
| Spezifischer Synthesegasbedarf | -1,7 |
| Verdichterleistung | -5,6 |
| Wärmetauscherleistung | -4,0 |
| Katalysatorvolumen | -17,1 |

Im Beispiel 1 werden gegenüber dem Vergleichsverfahren in allen genannten Verfahrensgrößen Einsparungen erzielt. Während der spezifische Synthesegasbedarf um 1,7 % geringfügig abnimmt, sinken die Verdichter- und die Wärmetauscherleistung um 5,6 % bzw. 4,0 %. Eine deutliche Abnahme ist mit 17,1 % auch beim benötigten Katalysatorvolumen und beim Kreislaufverhältnis mit 31,8 % zu verzeichnen. Dies entspricht einem Verhältnis von Kreislaufstrom zu Frischgasstrom von rund 70 % desjenigen beim Betrieb der Anlage ohne Nachreaktor gemäß Vergleichsverfahren.

### Beispiel 2: Synthesekreislauf mit einstufigem Synthesereaktor (wassergekühlt) + Nachreaktor (wassergekühlt)

| **Kenngröße** | **Relative Änderung / %** |
|---|---|
| Kreislaufverhältnis | -31,8 |
| Spezifischer Synthesegasbedarf | -0,4 |
| Verdichterleistung | -9,0 |
| Wärmetauscherleistung | +4,9 |
| Katalysatorvolumen | -57,6 |

Auch beim Beispiel 2 werden gegenüber dem Vergleichsverfahren bezüglich des spezifischen Synthesegasbedarfs und der Verdichterleistung Einsparungen erzielt. Die Wärmetauscherleistung steigt dagegen um 4,9 %. Besonders deutlich ist mit 57,6 % die erzielte Einsparung hinsichtlich des benötigten Katalysatorvolumens. Wie beim oberen Beispiel reduziert sich das Kreislaufverhältnisses um 31,8 %, wiederum entsprechend einem Verhältnis von Kreislaufstrom zu Frischgasstrom von rund 70 % desjenigen beim Betrieb der Anlage ohne Nachreaktor gemäß Vergleichsverfahren.

### Bezugszeichenliste

- 1: Leitung
- 2: Verdichter
- 3: Leitung
- 4: Wärmetauscher
- 5: Leitung
- 6: Synthesereaktor(en)
- 7: Leitung
- 8: Wärmetauscher
- 9: Leitung
- 10: Abscheider
- 11 - 14: Leitung
- 15: Verdichter
- 16 - 17: Leitung
- 18: Verdichter
- 19: Leitung
- 20: Wärmetauscher
- 21: Leitung
- 22: Nachreaktor
- 23: Leitung
- 24: Wärmetauscher
- 25: Leitung
- 26: Abscheider
- 27 - 30: Leitung
- 31: Verdichter
- 32: Leitung

## Patentansprüche

1. Verfahren zur Herstellung von Methanol aus einem Wasserstoff und Kohlenstoffoxide enthaltenden Synthesegas mit hohem Anteil an Inertkomponenten, wobei man
(a) das Synthesegas durch mindestens einen Synthesereaktor leitet, in welchem ein Teil der Kohlenstoffoxide katalytisch zu Methanol umgesetzt wird,
(b) aus dem erhaltenen, Synthesegas, Inertkomponenten und Methanoldampf enthaltenden Gemisch das Methanol abtrennt,
(c) das von Methanol befreite Gemisch in einen Kreislaufstrom und einen Spülstrom auftrennt,
(d) den Kreislaufstrom zum mindestens einen Synthesereaktor zurückführt und auf diese Weise einen Synthesekreislauf bildet,
(e) den Kreislaufstrom vor erneuter Aufgabe auf den Synthesereaktor mit einem Wasserstoff und Kohlenstoffoxide enthaltenden Frischgasstrom vereinigt, **dadurch gekennzeichnet, dass**
(f) der Spülstrom einem außerhalb des Synthesekreislaufs angeordneten Nachreaktor zugeführt wird, in dem ein weiterer Teil des Wasserstoffs und der Kohlenstoffoxide katalytisch zu Methanol umgesetzt werden,
(g) aus dem erhaltenen, Synthesegas, Inertkomponenten und Methanoldampf enthaltenden Gemisch weiteres Methanol abgetrennt wird.

2. Verfahren nach Anspruch 1, **dadurch gekennzeichnet, dass** das Verhältnis von Kreislaufstrom zu Frischgasstrom kleiner ist als bei einem Verfahren zur Herstellung von Methanol mit den Merkmalen 1 (a) bis 1 (e).

3. Verfahren nach Anspruch 1 und 2, **dadurch gekennzeichnet, dass** das Verhältnis von Kreislaufstrom zu Frischgasstrom 10 bis 90 %, besonders bevorzugt von 50 bis 80 % desjenigen eines Verfahrens zur Herstellung von Methanol mit den Merkmalen 1 (a) bis 1 (e) beträgt.

4. Verfahren nach einem der vorhergehenden Ansprüche, **dadurch gekennzeichnet, dass** das Verhältnis von Kreislaufstrom zu Frischgasstrom in Abhängigkeit von dem Anteil an Inertkomponenten im Frischgasstrom geregelt wird.

5. Verfahren nach einem der vorhergehenden Ansprüche, **dadurch gekennzeichnet, dass** der in Schritt 1 (g) erhaltene Gasstrom aus dem Verfahren entfernt wird.

6. Verfahren nach einem der vorhergehenden Ansprüche, **dadurch gekennzeichnet, dass** das in Schritt 1 (g) erhaltene Methanol der weiteren Produktaufarbeitung zugeführt wird.

7. Verfahren nach einem der vorhergehenden Ansprüche, **dadurch gekennzeichnet, dass** das in Schritt 1 (g) erhaltene Methanol vor Zuführung zur Produktaufarbeitung mit dem in Schritt 1 (b) erhaltenen Methanol vereinigt wird.

8. Verfahren nach einem der vorhergehenden Ansprüche, **dadurch gekennzeichnet, dass** der oder die Synthesereaktoren wassergekühlt, gasgekühlt oder adiabat betrieben werden.

9. Verfahren nach einem der vorhergehenden Ansprüche, **dadurch gekennzeichnet, dass** zwei Synthesereaktoren 6 vorhanden sind, wobei der erste Synthesereaktor wassergekühlt und der zweite Synthesereaktor gasgekühlt betrieben wird.

10. Verfahren nach einem der vorhergehenden Ansprüche, **dadurch gekennzeichnet, dass** der Nachreaktor wassergekühlt, gasgekühlt oder adiabat betrieben wird.

11. Anlage zur Herstellung von Methanol aus einem Wasserstoff und Kohlenstoffoxide enthaltenden Synthesegas mit hohem Anteil an Inertkomponenten, insbesondere zur Durchführung eines Verfahrens nach einem der vorhergehenden Ansprüche, mit einem oder mehreren Synthesereaktoren 6, in welchem ein Teil der Kohlenstoffoxide katalytisch zu Methanol umgesetzt wird, einem Abscheider 10 zur Abtrennung des Methanols von dem Synthesegas, Leitungen 12, 14, 16 zur Rückführung eines Synthesegas-Kreislaufstroms zum mindestens einen Synthesereaktor, und Leitungen 13, 19, 21 zum Abführen eines Spülstroms, **gekennzeichnet durch** einen mit Leitung 21 verbundenen, außerhalb des Synthesekreislaufs angeordneten Nachreaktor 22, in dem ein weiterer Teil des Wasserstoffs und der Kohlenstoffoxide katalytisch zu Methanol umgesetzt werden, und einen Abscheider 26 zur Abtrennung weiteren Methanols aus dem erhaltenen, Synthesegas, Inertkomponenten und Methanoldampf enthaltenden Gemisch.

12. Verfahren zur Umrüstung einer bestehenden Anlage zur Herstellung von Methanol gemäß Merkmal (a) bis (e) des Anspruchs 1 vom Betrieb mit inertenarmem Synthesegas für den Betrieb mit inertenreichem Synthesegas, insbesondere für die Durchführung eines Verfahrens nach Anspruch 1 bis 10, **dadurch gekennzeichnet, dass**
- dem Spülstrom ein außerhalb des Synthesekreislaufs angeordneter Nachreaktor nachgeschaltet wird, aus dessen Produktgemisch weiteres Methanol abgetrennt wird und
- die Anlage mit einem Verhältnis von zurückgeführtem Synthesegasstrom zum Frischgasstrom betrieben wird, das kleiner ist als beim Betrieb der Anlage mit inertenarmem Synthesegas.

## Claims

1. A method for the production of methanol from a synthesis gas containing hydrogen and carbon oxides with a high content of inert components, wherein
(a) the synthesis gas is passed through at least one synthesis reactor, in which a part of the carbon oxides is catalytically converted to methanol,
(b) the methanol is separated from the obtained mixture containing synthesis gas, inert components and methanol vapor,
(c) the mixture liberated from methanol is separated into a cycle stream and a purge stream,
(d) the cycle stream is recirculated to the at least one synthesis reactor and in this way forms a synthesis cycle,
(e) the cycle stream is combined with a fresh gas stream containing hydrogen and carbon oxides before again being charged to the synthesis reactor,
**characterized in that**
(f) the purge stream is supplied to a secondary reactor, being arranged outside of the synthesis cycle, in which a further part of the hydrogen and the carbon oxides is catalytically converted to methanol,
(g) further methanol is separated from the obtained mixture containing synthesis gas, inert components and methanol vapor.

2. The method according to claim 1, **characterized in that** the ratio of cycle stream to fresh gas stream is smaller than in a method for the production of methanol with the features 1(a) to 1 (e).

3. The method according to claims 1 and 2, **characterized in that** the ratio of cycle stream to fresh gas stream amounts to 10 to 90 %, particularly preferably from 50 to 80 % of that of a method for the production of methanol with the features 1(a) to 1(e).

4. The method according to any of the preceding claims, **characterized in that** the ratio of cycle stream to fresh gas stream is controlled in dependence on the content of inert components in the fresh gas stream.

5. The method according to any of the preceding claims, **characterized in that** the gas stream obtained in step 1 (g) is removed from the process.

6. The method according to any of the preceding claims, **characterized in that** the methanol obtained in step 1 (g) is supplied to the further product processing.

7. The method according to any of the preceding claims, **characterized in that** the methanol obtained in step 1 (g) is combined with the methanol obtained in step 1 (b) before being supplied to the product processing.

8. The method according to any of the preceding claims, **characterized in that** the one or more synthesis reactors are operated in a water-cooled, gas-cooled or adiabatic manner.

9. The method according to any of the preceding claims, **characterized in that** two synthesis reactors 6 are provided, wherein the first synthesis reactor is operated in a water-cooled manner and the second synthesis reactor in a gas-cooled manner.

10. The method according to any of the preceding claims, **characterized in that** the secondary reactor is operated in a water-cooled, gas-cooled or adiabatic manner.

11. A plant for the production of methanol from a synthesis gas containing hydrogen and carbon oxides with a high content of inert components, in particular for carrying out a method according to any of the preceding claims, comprising one or more synthesis reactors 6 in which a part of the carbon oxides is catalytically converted to methanol, a separator 10 for separating the methanol from the synthesis gas, conduits 12, 14, 16 for recirculating a synthesis gas cycle stream to at least one synthesis reactor, and conduits 13, 19, 21 for discharging a purge stream, **characterized by** a secondary reactor 22, being arranged outside of the synthesis cycle, connected with conduit 21, in which a further part of the hydrogen and the carbon oxides is catalytically converted to methanol, and a separator 26 for separating further methanol from the obtained mixture containing synthesis gas, inert components and methanol vapor.

12. A method for converting an existing plant for the production of methanol according to features (a) to (e) of claim 1 from the operation with synthesis gas low in inerts to the operation with synthesis gas rich in inerts, in particular for carrying out a method according to claims 1 to 10, **characterized in that**
- downstream of the purge stream a secondary reactor is provided, being arranged outside of the synthesis cycle, from whose product mixture further methanol is separated, and
- the plant is operated with a ratio of recirculated synthesis gas stream to fresh gas stream which is smaller than in operation of the plant with synthesis gas low in inerts.

## Revendications

1. Procédé de production de méthanol à partir d'un gaz de synthèse contenant de l'hydrogène et des oxydes de carbone et ayant une grande proportion de constituants inertes, dans lequel
(a) on envoie le gaz de synthèse dans au moins un réacteur de synthèse, dans lequel une partie des oxydes de carbone est transformée catalytiquement en méthanol,
(b) on sépare du gaz de synthèse obtenu un mélange contenant des constituants inertes et de la vapeur de méthanol,
(c) on sépare le mélange débarrassé de méthanol en un courant de circuit et en un courant d'entraînement,
(d) on retourne le courant de circuit au au moins un réacteur de synthèse et on forme ainsi un circuit de synthèse,
(e) on réunit le courant de circuit, avant de le recharger dans le réacteur de synthèse, à un courant de gaz frais contenant de l'hydrogène et des oxydes de carbone, **caractérisé en ce que**
(f) on envoie le courant d'entraînement à un post-réacteur à l'extérieur du circuit de synthèse, dans lequel on transforme catalytiquement en méthanol une autre partie de l'hydrogène et des oxydes de carbone,
(g) on sépare encore du méthanol du mélange contenant du gaz de synthèse obtenu, des constituants inertes et de la vapeur de méthanol.

2. Procédé suivant la revendication 1, **caractérisé en ce que** le rapport du courant de circuit au courant de gaz frais est plus petit que dans un procédé de production de méthanol ayant les caractéristiques 1(a) à 1(e).

3. Procédé suivant la revendication 1 ou 2, **caractérisé en ce que** le rapport du courant de circuit au courant de gaz frais représente de 10 à 90%, d'une manière particulièrement préférée de 50 à 80%, de celui d'un procédé de production de méthanol ayant les caractéristiques 1(a) à 1(e).

4. Procédé suivant l'une des revendications précédentes, **caractérisé en ce qu'**on règle le rapport du courant de circuit au courant de gaz frais en fonction de la proportion de constituants inertes dans le courant de gaz frais.

5. Procédé suivant l'une des revendications précédentes, **caractérisé en ce qu'**on élimine du procédé le courant de gaz obtenu au stade 1(g).

6. Procédé suivant l'une des revendications précédentes, **caractérisé en ce qu'**on envoie du méthanol obtenu au stade 1(g) à un autre retraitement de produit.

7. Procédé suivant l'une des revendications précédentes, **caractérisé en ce qu'**on réunit le méthanol obtenu au stade 1(g) au méthanol obtenu au stade 1(b) avant l'envoi au retraitement du produit.

8. Procédé suivant l'une des revendications précédentes, **caractérisé en ce qu'**on refroidit par de l'eau, on refroidit par du gaz ou on fait fonctionner adiabatiquement le ou les réacteurs de synthèse.

9. Procédé suivant l'une des revendications précédentes, **caractérisé en ce qu'**il y a deux réacteurs (6) de synthèse, le premier réacteur de synthèse fonctionnant avec un refroidissement par de l'eau et le deuxième réacteur de synthèse fonctionnant par un refroidissement par du gaz.

10. Procédé suivant l'une des revendications précédentes, **caractérisé en ce qu'**on fait fonctionner le post-réacteur avec refroidissement par de l'eau, avec refroidissement par du gaz ou adiabatiquement.

11. Installation de production de méthanol à partir d'un gaz de synthèse contenant de l'hydrogène et des oxydes de carbone et ayant une grande proportion de constituants inertes, notamment pour effectuer un procédé suivant l'une des revendications précédentes, comprenant un ou plusieurs réacteurs (6) de synthèse, dans lequel on transforme catalytiquement en méthanol une partie des oxydes de carbone, un séparateur (10) pour séparer le méthanol du gaz de synthèse, des conduits (12, 14, 16) de retour d'un courant de circuit de gaz de synthèse à au moins un réacteur de synthèse et des conduits (13, 19, 21) d'évacuation d'un courant d'entraînement, **caractérisée par** un post-réacteur (22), qui est relié par un conduit (21), qui est à l'extérieur du circuit de synthèse et dans lequel on transforme catalytiquement en méthanol une autre partie de l'hydrogène et des oxydes de carbone, et un séparateur (26) pour séparer encore du méthanol du mélange contenant le gaz de synthèse obtenu, des constituants inertes et de la vapeur de méthanol.

12. Procédé pour transformer une installation existante de production de méthanol suivant les caractéristiques (a) à (e) de la revendication 1, du fonctionnement avec du gaz de synthèse pauvre en inerte au fonctionnement avec du gaz de synthèse riche en inerte, notamment pour effectuer un procédé suivant les revendications 1 à 10, **caractérisé en ce que**
- on monte un post-réacteur à l'intérieur du circuit de synthèse en aval du courant de balayage, du mélange de produit duquel on sépare encore du méthanol et
- on fait fonctionner l'installation avec un rapport du courant de gaz de synthèse recyclé au courant de gaz frais, qui est plus petit que dans le fonctionnement de l'installation avec du gaz de synthèse pauvre en inerte.
